# EUROPEAN PATENT SPECIFICATION

(11) **EP 1 925 294 B1**
(45) Date of publication and mention of the grant of the patent: **08.04.2015**
(21) Application number: 07254580.9
(22) Date of filing: 26.11.2007
(51) Int. Cl.: A61K 9/127, A61K 9/19, A61K 47/02, A61K 47/36, A61K 9/14, A61K 9/16, A61K 9/48, A61K 9/20

(54) **Methods and formulations for enhancing the absorption and decreasing the absorption variability of orally administered drugs, vitamins and nutrients**
Verfahren und Formulierungen zur Verbesserung der Absorption und Verringerung der Absorptionsvariabilität von oral verabreichten Arzneimitteln, Vitaminen oder Nährstoffen
Procédés et préparations pour améliorer l'absorption et pour diminuer la variabilité d'absorption de médicaments, vitamines et agents nutritifs administrés par voie orale

(30) Priority: 27.11.2006 US 563356
(43) Date of publication of application: 28.05.2008
(73) Proprietor: Zomanex, LLC, Chesterfield, MO 63017 (US)
(72) Inventor: Spilburg, Curtis A. Zomanex, LLC, Chesterfield, MO 63017 (US)
(74) Representative: Wood, Graham

(56) References cited:
- EP-A- 0 296 845
- WO-A-03/094891
- US-A- 5 202 126
- US-A- 5 290 562
- US-A- 5 415 869

## Description

### FIELD OF THE INVENTION

This invention relates to a general method for enhancing the bioavailability of hydrophobic drug active compounds, using naturally-occurring formulation ingredients that are present in the diet. Specifically, this invention is especially useful as a general formulation method for the delivery of drugs in liquid or dry form that heretofore have produced variable pharmacological responses, which are indicative of poor bioavailability.

### BACKGROUND OF THE INVENTION

Oral drug delivery, the preferred method of administration for most people, remains a subject of intense pharmaceutical and biochemical investigation since the mechanism(s) of drug absorption in the small intestine is largely unknown. It is generally believed that two processes control the amount of drug that is absorbed. First, a high concentration of the active substance at the intestinal membrane surface will enhance cellular absorption (Fick's Law) and, since cells function in an aqueous environment, enhancing the water solubility of a drug increases its concentration at the locus of absorption. However, even though greater water solubility may be expected to enhance the bioavailability of drugs, this is frequently not the case due to a second, competing process that affects the overall absorption process. Thus, the absorptive cell membrane is composed mainly of lipids that prevent the passage of hydrophilic water-soluble compounds, but which are highly permeable to lipid soluble substances. Therefore, the design of bioavailable drugs must balance two opposing forces. On the one hand, a drug that is very hydrophilic may have a high concentration at the cell surface but it may be impermeable to the lipid membrane. On the other hand, a hydrophobic drug that may easily "dissolve" in the membrane lipids may be virtually insoluble in water producing a very low concentration of the active substance at the cell surface.

The intestinal plasma membrane lines the lumen of the upper gut and is the first absorptive surface to be permeated by most nutrients, foodstuffs and drugs. As part of the digestive process, the apical side of the cell is exposed to a complex milieu consisting of pancreatic enzymes, bile and partially digested food from the stomach. Drug absorption does not occur in isolation. Since most drugs are lipophilic, their absorption takes place along with or in competition with that for other lipophilic molecules, such as cholesterol, fat-soluble vitamins, oils and fatty acids. The small intestine is densely covered with villi and microvilli, which greatly enhance the area available for absorption (250 m²), favoring the uptake of even poorly soluble substances, Moreover, the cell surface is also covered with heparin, a negatively charged polysaccharide that tightly binds lipolytic enzymes, such as cholesterol esterase and triglyceride lipase, providing a locus of hydrolytic activity virtually contiguous with the absorptive surface (Bosner MS, et al., Proc Natl Acad Sci 85: 7438-7442,1989). This tight binding interaction ensures a high level of lipolytic activity even when the pancreas is not secreting enzymes.

The combination of lipolytic enzymes, bile components and a large intestinal absorption surface provides an environment in which virtually all food is absorbed (Armand M et al., Am J Physiol 271: G172-G183, 1996). While the above-mentioned processes are extremely efficient, the same is not true for certain chemically complex lipids, such as cholesterol, plant sterols, fat soluble vitamins, naturally occurring dietary nutrients and drugs. For example, unlike other fats, cholesterol from the diet or the bile is not completely absorbed, and human cholesterol absorption has a mean value of 55%, with considerable variation from individual to individual (Bosner MS et al., J Lipid Res 40: 302-308, 1999). Over the past twenty years, much progress has been made in delineating and understanding the biochemical processes that are used for the absorption of this class of compound. Even though compounds like cholesterol are not thought of as "drugs," the pathways used for their absorption may be common to other substances end they may provide insight into better ways for drug delivery.

A central feature of this new understanding of lipid absorption is the identification, isolation and dynamic interplay of individual intestinal proteins in the overall absorption process. For example, Niemann-Pick C1-Like protein 1 (NPC1L1) and scavenger receptor class B proteins were recently shown to be responsible for cholesterol uptake by the small intestine and a drug that inhibits cholesterol absorption, ezetimibe, binds to and blocks the action of both these proteins (Davis HR et al., Biol Chem 279: 33586-33592, 2004). In addition to proteins involved in sterol absorption, there are others that promote its excretion. Thus, the ATP-binding cassette transporters, ABCG5 and ABCG8, present in the apical membrane of the enterocyte actively excrete sterols back to the intestinal lumen (van Meer G et al., FEBS Letters 580: 1171-1177, 2006). The net absorption of sterol is then determined, at least in part, by the interplay of an influx transporter (NPC1L1) and an efflux transporter (ABCG5 or ABCG8). This type of motif also occurs with many drugs through the action of the efflux transporters, multidrug resistance-association protein 1 and 2 (MDR1 and MDR2), located in high concentration on the villus tip of the apical surface of the brush border membrane, where they can serve as a barrier for the intestinal absorption of numerous drug substrates (Pang KS, Drug Metab Disp 31: 1507-1519, 2005).

In addition to transporters and exporters, the small intestinal cell also contains a number of binding proteins, such as retinol binding protein, fatty acid binding protein and sterol carrier protein, which are used to shuttle complex lipids throughout the cellular cytosol and thereby enhance the uptake process (Tso P et al., Biochem Soc Trans 32: 75-78, 2004). One of these, fatty acid binding protein, possesses a large binding site and it has recently been shown that a number of drugs including ibuprofen, bezafibrate and nitrazepam can be accommodated in this binding site (Velkov T et al., J Biol Chem 280: 17769-17776,2005). Since the drug competes with fatty acid for this site, this may explain why some drugs are poorly absorbed after consumption of a fatty meal. The existence of and putative function of these proteins show that structurally complex lipids rarely appear "free" in solution, but they are shuttled from place to place as a component of a larger structure such as a protein transporter or binding protein within the cell or, far transport outside the cell, in a "particle" that contains phospholipid, cholesterol ester, triglyceride and apoproteins, such as chylomicrons or low-density lipoproteins (Fielding PE et al., In Biochemistry of Lipids, Lipoproteins and Membranes. eds. DE Vance and J Vance. Elsevier, 1991, pp 427-458).

Given the fact that the drug absorption process is dependent on a constellation of physiological factors, it is not surprising that children (especially neonates and infants) do not show the same pharmacokinetics and pharmacodynamics as that found in adults (Niderhauser, VP, Nurse Pract 22: 6-28, 1997). Thus, in neonates and infants, the pancreas is not fully developed so the level of lipolytic activity in the duodenum is lower than that found in adults. For example, at an age of one month, the newborn pancreas does not produce measurable amounts of lipase or amylase, while considerable trypsin activity is detected at all ages (Lebenthal, B et al., Pediatrics 66: 556-560, 1980). Moreover, the bile acid concentration in infant duodenal juice is much lower than that found in adults and may be less than the amount required for good emulsification of fatty substrates and lipid drugs (Murphy, GM et al., Gut 15: 151-163, 1974). The absence of a full range of lipolytic enzymes and the suboptimal bile salt concentration may provide an explanation for low fat absorption during the first year of life and also may support the use of different drug delivery systems and formulations for this special group compared to those used for adults (Norman A. et al., Acta Paediat Scand 61: 571-76, 1972).

Regardless of chronological age, the body has chemical structures for moving complex lipids from place to place, and this type of motif is also used to aid in the movement of the same compounds from the intestinal humen to the intestinal cell for subsequent absorption. This possibility has been recognized by the pharmaceutical industry for sometime, and a variety of self-emulsifying drug delivery systems have been devised that package the drug in a variety of lipids and surfactants that provide a dispersible matrix when the combination is ingested (Devani, M et al., J Pharm Pharmacol 56, 307-316, 2004). Alternatively, it has been suggested that formulations that are patterned after the lipid composition of digestion phases may provide insight into better ways to solubilize water insoluble drugs (Porter CJH, et al., J Pharm Sci 93: 1110-1121, 2004). While these studies have demonstrated the importance of the digestion process as a guide or template for drug absorption, the approach is empirical requiring exhaustive studies for each drug. Moreover, their approach is focused more on the physical chemistry of solubilization than on the biochemistry of absorption so they provide little additional insight into the molecular events that are an integral and obligatory part of the absorption process.

Another delivery strategy has been the use of liposomes as an encapsulation vehicle for a variety of drugs for different delivery routes, including oral, parenteral and transdermal (Cove, G and Paltauf, F., eds., Phospholipids: Characterization, Metabolism. and Novel Biological Applications, pp. 67-79,126-133, AOCS Press, Champaign, IL, 1995). This method requires amphiphiles, compounds that have a hydrophilic or polar end group and a hydrophobic or non-polar end group, such as phospholipid, cholesterol, glycolipid or a number of food-grade emulsifiers or surfactants. When amphiphiles are added to water, they form lipid bilayer structures (liposomes) that contain an aqueous core surrounded by a hydrophobic membrane. This novel structure can deliver water insoluble drugs that are "dissolved" in its hydrophobic membrane or, alternatively, water soluble drugs can be encapsulated within its aqueous core. This strategy has been employed in a number of fields. For example, liposomes have been used as drug carriers since they are rapidly taken up by the cell and, moreover, by the addition of specific molecules to the liposomal surface they can be targeted to certain cell types or organs, an approach that is typically used for drugs that are encapsulated in the aqueous core. For cosmetic applications, phospholipids and lipid substances are dissolved in organic solvent and, with solvent removal, the resulting solid may be partially hydrated with water and oil to form a cosmetic cream or drug-containing ointment. Finally, liposomes have been found to stabilize certain food ingredients, such as omega-3 fatty acid-containing fish oils to reduce oxidation and rancidity (Haynes et al, U.S. Patent 5,139,803).

Even though liposomes provide an elegant method for drug delivery, their use has been limited by cumbersome preparation methods, inherent instability of aqueous preparations and low drug loading capacity for solid, oral preparations. The utility of a dried preparation to enhance the stability and shelf life of the liposome components has long been recognized, and numerous methods have been devised to maintain the stability of liposomal preparations under drying conditions. [Schneider (U.S. Patent 4,229,360), Rahman et al, (4,963,362). Vanlerberghe et al. (U.S. Patent 4,247,411), Payne et al. (U.S. Patents 4,744,989 and 4,830,858)]. The goal of all these methods is to produce a solid that can be re-hydrated at a later time to form liposomes that can deliver a biologically active substance to a target tissue or organ, Surprisingly, there have been only two reports that use the dried liposome preparations themselves, with no intermediate hydration, as the delivery system. Ostlund, U.S. Patent 5,932,562 teaches the preparation of solid mixes of plant sterols for the reduction of cholesterol absorption. Plant sterols or plant stanols are premixed with lecithin or other amphiphiles in organic solvent, the solvent removed and the solid added back to water and homogenized. The emulsified solution is dried and dispersed in foods or compressed into tablets or capsules. In this case, the active substance is one of the structural components of the liposome itself (plant sterol) and no additional biologically active substance was added. Manzo et al. (U.S. patent 6,083,529) teach the preparation of a stable dry powder by spray drying an emulsified mixture of lecithin, starch and an anti-inflammatory agent. When applied to the skin, the biologically active moiety is released from the powder only in the presence of moisture. Neither Ostlund nor Manzo suggest or teach the use of sterol, and lecithin and a drug active, all combined with a non-polar solvent and then processed to provide a dried drug carrying liposome of enhanced delivery rates.

Substances other than lecithin have been used as dispersing agents. Following the same steps (dissolution in organic solvent, solvent removal, homogenization in water and spray drying) as those described in U.S. Patent 5,932,562, Ostlund teaches that the surfactant sodium steroyl lactylate can be used in place of lecithin (U.S. Patent 6,063,716). Burruano et al. (U.S. Patents 6,054,144 and 6,110,502) describe a method of dispersing soy sterols and stanols or their organic acid esters in the presence of a mono-functional surfactant and a poly-functional surfactant without homogenization. The particle size of the solid plant-derived compounds is first reduced by milling and then mixed with the surfactants in water. This mixture is then spray dried to produce a solid that can be readily dispersed in water. Similarly, Bruce et al. (U.S. Patent 6,242,001) describe the preparation of melts that contain plant sterols/stanols and a suitable hydrocarbon. On cooling these solids can be milled and added to water to produce dispersible sterols. Importantly, none of these methods anticipate the type of delivery method described here as a means to deliver hydrophobic, biologically active compounds.

None of the previous art suggests or teaches methods to enhance the uptake of a drug/sterol/amphilphile combination at a drug loading capacity that would lead to a commercially viable drug delivery system. The stability and ultimate use of liposomal preparations have been shown to depend on the ratio of lecithin to the sterol drug combination. Thus, in order to form creams and parenteral liposomal preparations, previous work focused on the preparation of dispersions containing small liposomal particles (less than 1 µm) by maintaining a high ratio of lecithin to the other components. This prejudice was shown by the requirement that the sum of the drug and the sterol present should not exceed about 25% and preferably about 20% of the total lipid phase present Hence, the previous art teaches a ratio of lecithin to the sum of the sterol and drug components of at least 4.0, and preferably 5.0 [Perrier et al., U.S. Patent 5,202,126 (c2, line 45), Meybeck & Dumas, U.S. Patent 5,290,562 (c3, line 29)]. Moreover, the purpose of this requirement was to maintain liposomal "quality," which was achieved with a small particle size in order to enhance the stability of the dispersion for the intended uses contained therein [Perrier et al., U.S. Patent 5,202,126 (c4, line 61)]. Departure from this preferred ratio produced sediment which "detracts from the stability of the liposomes" [Perrier et al., U.S. Patent 5,202,126, (c5, line 10)].

In contrast, for the preparation of oral dosage forms it was shown that a superior preparation contained a ratio of the sterol drug combination to amphiphile of 0.2 to 3.0. (Spilburg, patent application, S.N. 11/291,126, November 30, 2005). This combination produces a delivery system with the following useful and novel advantages: a dispersed solution that can be dried and re-hydrated to produce a dispersion of particles that is similar to that of the dispersion from which it was derived; high drug loading capacity by minimizing the amount of amphiphile in the mix; an emulsion that is stable to conventional drying methods without the addition of large amounts of stabilizers. The dried solid so manufactured can be easily compacted in a tablet and capsule to render the hydrophobic drug bioavailable on ingestion and easily deliverable in a pharmaceutical format. Moreover, while the previous work described above focused on the delivery of solid, crystalline drugs, this work extends the utility of this method to show that the same high drug loading can be achieved with oils.

Because it is generally believed that remove of water from liposomes may produce a preparation that has lost its biological activity, there has been much work on adjusting conditions for optimizing the integrity of the liposome on dehydration. On the other hand, there has been little work on methods to enhance the stability of such preparations when used in oral dosage forms. This absence of art is primarily due to the fact that most work in this area has focused on the formation of gels and creams from the dried liposomal material, a process that does not utilize extreme physical chemical conditions. On the other hand, passage and dissolution of a tablet or capsule containing a dried liposomal preparation through the highly acidic conditions found in the stomach places much greater stress on maintaining the integrity of this delivery system before it reaches its site of action in the small intestine. Therefore, as shown in this work with oral drug delivery, additional additives such as buffers or antacids may be required to derive the full absorptive benefit of the liposomal preparation.

An object of the invention is to improve the following properties of a hydrophobic drug (oil or crystalline) by combining it with sterols and a combination of amphiphiles, surfactants or emulsifiers: (1) increase the amount of absorption (area under the curve), (2) improve the variability of absorption and (3) provide a powder formulation for a variety of delivery formats - tablets, chewable tablets, capsules, food additives and liquids.

### SUMMARY OF THE INVENTION

A general method is provided for enhancing the bioavailability of hydrophobic, poorly water soluble compounds and drugs that are either crystalline or oils. It employs the following steps:
(a) An amphiphile or a blend of amphiphiles, such as lecithin and one of its derivatives, a sterol (preferably a plant-derived sterol and most preferably a reduced plant-derived sterol) and the drug are mixed in a non-polar solvent (preferably ethyl acetate or heptane) at its boiling point.
(b) A solid is collected after the solvent is driven off at elevated temperature to maintain the solubility of all the components.
(c) The solid is broken into small pieces and dispersed with vigorous stirring in water at a temperature that is less than the decomposition temperature of one of the components or the boiling point of water, whichever is lower.
(d) The milky solution is passed through a Gaulin Dairy Homogenizer (or suitable equivalent) operating at maximum pressure; and thereafter
(e) a suitable drying aid is added (starch, silicon dioxide or suitable equivalent), and then the milky solution is spray dried or lyophilized to produce a solid that can be incorporated into tablets or capsules, providing the appropriate excipients are added.

In another method, the amphiphile, plant sterols and active drug are mixed in the presence of an organic solvent such as hexane or ethyl acetate, the solvent removed and the solid compressed and extruded for the formation of tablets and capsules.

The formulation method described herein contains a minimum of three components, an emulsifier(8), a sterol and a hydrophobic active or drug compound. The final dosage form may also contain a variety of excipients to aid in processing and to maintain liposomal stability when given as an oral dosage form.

### BRIEF DESCRIPTION OF THE DRAWINGS

Figure 1 shows in graph format, progesterone absorption data for dogs of Example 1.
Figure 2 shows in graph form data of Example 2.
Figure 3 shows in graph form data of Example 3.
Figure 4 shows in graph form data of Example 4.

### DETAILED DESCRIPTION OF A PREFERRED EMBODIMENT

Numerous amphiphilic emulsifiers have been described, but since this invention contemplates pharmaceutical application only those compounds that have been approved for human use are acceptable. A preferred emulsifier is lecithin derived from egg yolk, soy beans or any of its chemically modified derivatives, such as lysolecithin. Lecithin is not only an excellent emulsifier and surfactant, it also has many health benefits that are beneficial when used as the contemplated pharmaceutical formulation agent described here [Cevc, G. and Paltauf, F., eds., Phospholipids: Characterization, Metabolism, and Novel Biological Applications, pp. 208-227 AOCS Pres, Champaign, IL, 1995]. While many grades and forms are available, do-ofled lecithin produces the most consistent results. Typical commercially available examples are Ultralec P, Ultralec F and Ultralec G (Archer Daniels Midland, Decatur, IL) or Precept 8160, a powdered, enzyme-modified lecithin (Solae, Fon Wayne, IN).

Other emulsifiers can be successfully used including, but not limited to mono and diglycerides, diacetyltartaric acid esters of mono and diglycerides, monoglyceride phosphate, acetylated monoglycerides, ethoxylated mono and diglycerides, lactylated monoglycerides, propylene glycol esters, polyglycerol esters, polysorbates, sorbitan esters, sodium and calcium stearoyl lacrylate, succinylated monoglycerides, sucrose esters of fatty acids, fatty alcohols, sodium salts of fatty acids. In certain instances, combinations of these emulsifiers may also be used.

A variety of sterols and their ester derivatives can be added to the emulsifier(s) to enhance the aqueous dispersibility in the gut in the presence of bile salts and bile phospholipid. While cholesterol has frequently been used for this purpose, its absorption can lead to elevated LDL-cholesterol levels, making it a poor choice for the pharmaceutical applications contemplated here. Plant-derived sterols, especially those derived from soy and tall oil, are the preferred choice since they have been shown to lower LDL-cholesterol and they are considered to be Safe (Jones PJH et al., Can J. Physiol Pharmacol 75: 227-235,1996). Specifically, this invention contemplates the use of mixtures including, but not limited to sitosterol, campesterol, stigmasterol and brassicasterol and their corresponding fatty acid esters prepared as described elsewhere (Wester I., et al., "Stanol Composition and the use thereof", WO 98/06405). The reduced forms of the above-mentioned sterols and their corresponding esters are the most preferred, since they also lower human LDL-cholesterol and their absorption is from five- to ten-fold less than that of their non-reduced counterparts (Ostlund RE et al., Am. J. of Physiol, 282: E 911-E916, 2002; Spilburg C et al., J Am Diet Assoc 103: 577-581, 2003).

Hydrophobic drugs and potential drugs may be selected from any therapeutic class including but not limited to anesthetics, anti-asthma agents, antibiotics, antidepressants, anti-diabetics, anti-epileptics, anfi-funpls, anti-gout, anti-neoplastics, anti-obesity agents, anti-protozoals, anti-phyreties, anti-virals, anti-psychotics, calcium regulating agents, cardiovascular agents, corticosteroids, diuretics, dopaminergic agents, gastrointestinal agents, hormones (peptide and non-peptide), immunosuppressants, lipid regulating agents, phytoestrogens, prostaglandins, relaxants and stimulants, vitamins/nutritionals and xanthines. A number of criteria can be used to determine appropriate candidates for this formulation system, including but not limited to the following: drugs or organic compounds that are known to be poorly dispersible in water, leading to long dissolution times; drugs or organic compounds that are known to produce a variable biological response from dose to dose or; drugs that are oils that are difficult to deliver in a conventional tablet or capsule delivery system or, drugs or organic compounds that have been shown to be preferentially soluble in hydrophobic solvent as evidenced by their partition coefficient in the octanol water system; or drugs that are preferentially absorbed when consumed with a fatty meal. In addition to these components, other ingredients may be added that provide beneficial properties to the final product, such as vitamin E to maintain stability of the active species.

All the components are dissolved in a suitable non-polar organic solvent, such as chloroform, dichloromethane, ethyl acetate, pentane, hexane, heptane or supercritical carbon dioxide. The choice of solvent is dictated by the solubility of the components and the stability of the drug at the temperature of the solvent. The preferred solvents are non-chlorinated and for heat stable compounds, heptane is the most preferred solvent because of its high boiling point, which increases the overall solubility of all the components.

The weight ratio of the components in the final mixture depends on the nature of the hydrophobic compound, but regardless of its structure or other properties the goal is to produce an emulsified mixture of drug, sterols and amphiphile so that the amount of amphiphile in the system is minimized relative to the other two components. To achieve this end, the ratio of amphiphile to the drug sterol combination is less than 3.0. It can range from 0.2 to 10.0; is preferably within the range of 0.10 to 3.0; and most preferred at 1.5. Sufficient amphiphile must be present to allow emulsification such that the ratio of amphiphile to the sterol drug combination is at least 0.1 or greater.

After all the components are dissolved at the desired ratio in the appropriate solvent, the liquid is removed at elevated temperature to maintain the solubility and stability of all the components. Residual solvent can be removed by pumping under vacuum. Alternatively, the solvent can be removed by atomization as described in U.S. Patents 4,508,703 and 4,621,023. The solid is then added to water at a temperature that is less than the decomposition temperature of one of the components or the boiling point of water, whichever is lower. The mixture is vigorously mixed in a suitable mixer to form a milky solution, which is then homogenized, preferably with a sonicator, Gaulin dairy homogenizer or a microfluidizer. The water is then removed by spray drying, lyophilization or some other suitable drying method. Before drying, it is helpful but not necessary, to add maltrin, starch, silicon dioxide, calcium silicate or sodium croscarmellose to produce a flowable powder that has more desirable properties for filling capsules, compression into tablets or addition to certain medical foods. The addition of a suitable antacid, such as calcium carbonate or the like, to the powder at a weight per cent of 0.5 to 10.0 stabilizes and/or activates the components in the blend to produce a superior product. For some blends, either wet or solid granulation produces a superior solid with a greater bulk density.

The dried liposomal blend described above is the starting point for a variety of flexible delivery systems described below. Since the key components of the powdered formulation system are compounds that are an integral result of the digestive process, they are compatible with food delivery systems that can be especially designed for children and the elderly. The powdered drug/plant sterol/lecithin blend described above can be easily dispersed in milk or other beverages for convenient delivery to neonates and infants. Moreover, the absence of pancreatic lipolytic activity and low concentrations of bile salt are not an impediment to drug absorption since the drug is packaged in a system that contains components that are the end product of the digestive process. This is of special importance for neonates and adults with pancreatic insufficiency, such as cystic fibrosis patients. In summary, the proposed formulation system provides a seamless transition from neonates - powder dispersed in milk - to children - powder compressed in a chewable tablet - to adults - powder compressed in a conventional tablet or capsules - to the elderly - powder dispersed in beverages or other supplemented drinks.

There are other known methods that can be used to prepare tablets. After the components have been mixed at the appropriate ratio in organic solvent, the solvent can be removed as described above. The solid material so prepared can then be compressed at elevated pressure and extruded into a rope. The rope can be cut in segments to form tablets, This method is similar to that described in U.S. Patent 6,312,703, but the inventor did not recognize the importance of pre-mixing the components in organic solvent. While this previous method produces a tablet, the components may not be as freely dispersible in bile salt and phospholipid when they are not pre-mixed in organic solvent. Alternatively, the solid material that results from homogenization and spray drying can be compressed at high pressure and extruded to form a rope that can be cut into tablets.

The precise details of tableting technique are not a part of this invention, and since they are well-known they need not be described herein in detail. Generally pharmaceutical carriers which are liquid or solid may be used. The preferred liquid carrier is water, but milk can also be used especially for neonates and infants. Flavoring material may be included in the solutions as desired.

Solid pharmaceutical carriers such as starch, sugar, talc, mannitol and the like may be used to form powders. Mannitol is the preferred solid carrier. The powders may be used as such for direct administration to a patient, or instead, the powders may be added to suitable foods and liquids, including water, to facilitate administration.

The powders also may be used to make tablets, or to fill gelatin capsules. Suitable lubricants like magnesium stearate, binders such as gelatin, and disintegrating agents like sodium carbonate either alone or in combination with citric acid may be used to form the tablets.

While not precisely knowing why, and not wishing to be bound by any theory of operability, the fact is that for difficulty soluble drugs this composition and combination of steps achieved higher absorption and lower variability of absorption.

In the examples to follow, the novelty and utility of the method will be shown in both liquid and solid delivery systems that employ the formulation methods described above. The improvement in the uptake and its predictability will be shown by comparing the proposed formulation system to that available in the corresponding commercially available drug, To these ends, pharmacokinetic studies were performed in four naive beagle dogs with each drug dosed in a formulation system using a crossover design. For studies with progesterone, only male dogs were used while female dogs were used for all the others. All animal work was performed following procedures for animal care and housing that were in accordance with the Guide for the Care and Use of Laboratory Animals (Institute of Laboratory Animal Resources, Commission on Life Sciences, National Research Council, National Academic Press, 1996). Following a 16-hour fast, the animals were orally dosed with one of the formulations of the appropriate test article. Blood samples were drawn 0.5, 1.0, 1.5, 2.0, 4.0, 6.0, 8.0, 10.0 and 24 hours after dosing.

### EXAMPLE 1

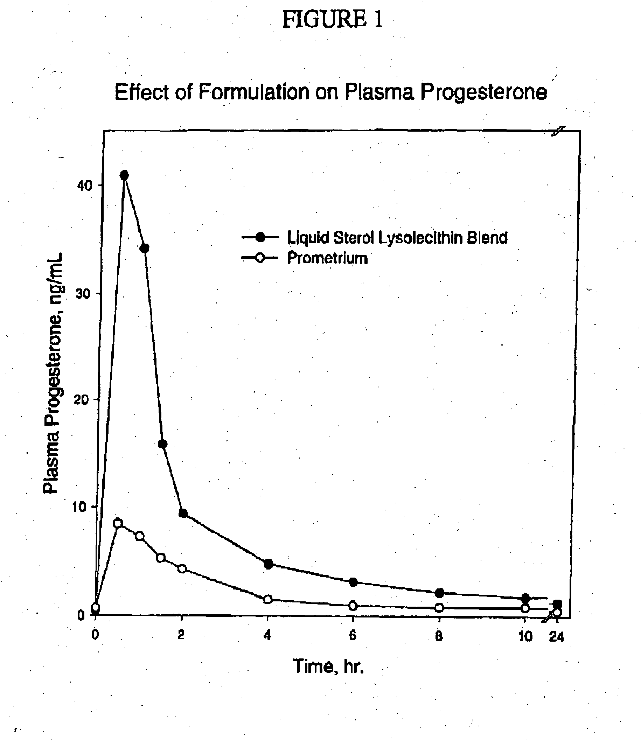

Progesterone (100 mg, LKT Laboratories, St. Paul, MN), soy sterols (100 mg, Archer Daniels Midland, Decatur, IL) and soy lysolecithin (300 mg, Solae Corporation, Fort Wayne IN) were dissolved in chloroform with gentle warming and the solvent was removed under a stream of nitrogen, followed by exhaustive pumping. On the day of the experiment, water (10 mL) was added to the glass tube and the mixture was sonicated for 30 seconds on ice using a Branson Digital Sonifier (Model S450D), equipped with a 1/8" tapered tip to form a milky emulsion, which was delivered to the dog with a syringe. Water was added to the syringe and the washing was administered to the dog. Commercial progesterone (Prometrium^{®}, Solvay Pharmaceuticals) was delivered in a capsule that contains micronized drug that is dispersed and partially dissolved in a peanut oil glycerin blend. After all the blood samples were collected and centrifuged, the plasma progesterone concentration was determined with a radioimmunoassay kit (Diagnostic Products Corporation) at each time point for each of the four dogs. As shown in Figure 1, there is a marked increase io progesterone absorption for the liquid formulation when compared to the oil dispersion used in Prometrium^{®}, reflected in a statistically significant 3-fold increase in the area under the curve of 99.8 ± 7.4 ng/mL h⁻¹ *vs.* 32.2 ± 7.1 ng/mL h⁻¹ (p = 0.006). In addition, for each formulation the coefficient of variation of the plasma progesterone concentration was determined at each time point for the four dogs. There was a statistically significant (p = 0.05) difference between the mean coefficient of variation calculated for all time points for Prometrium^{®} and the liquid lysolecithin/sterol/progesterone formulation of 32.7% and 18.7%, respectively. These data indicate that the liquid lysolecithin/sterol progesterone formulation also provides less variation in progesterone uptake than that provided by the conventional formulation.

### EXAMPLE 2

The liquid formulation method was also used to determine the effect of different emulsifiers and their combination on the uptake of progesterone. The following formulations were prepared by mixing the corresponding solids in chloroform followed by solvent removal.

| Progesterone | Sterols | Lecithin | Lysolecithin |
|---|---|---|---|
| 100 mg | 100 mg | 300 mg | 0 mg |
| 100 mg | 100 mg | 200 mg | 100 mg |
| 100 mg | 100 mg | 100 mg | 200 mg |

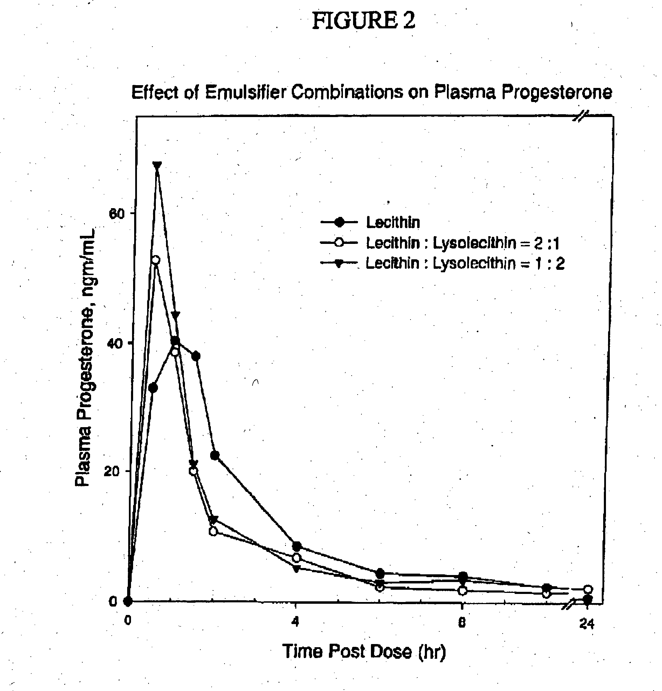

All subsequent steps were the same as those described in Example 1. As shown in Figure 2, the maximum concentration of plasma progesterone (Cₘₐₓ) decreases as the fraction of lecithin increases in the liquid formulation. This is accompanied by higher concentrations of plasma progesterone from 1.5 to 6 hours post dose than that found in the formulations that contain lysolecithin. This shows that combinations of emulsifiers can be used either to advantageously prolong the effect of drug (high lecithin content in the formulation) or to produce a faster onset of drug-related biological activity (high lysolecithin content in the formulation),

### EXAMPLE 3

### Sprionolactone was examined in three different formulation systems,

*Liquid Formulation.* A liquid formulation was prepared the same way as that described for progesterone in Example 1.

*Solid Formulation Derived From the Liquid Formulation.* Spironolactone (500 mg), plant sterols (500 mg) and lysolecithin (1500 mg) were added to each of three 30-mL glass tubes and dissolved in chloroform. Solvent was removed under nitrogen and the solid was kept under vacuum to remove residual solvent. Water (20 mL) was then added to each tube and the contents were sonicated on ice using a Branson Digital Sonifier (Model S450D), equipped with a 1/8" tapered tip. Initially, the sample was sonicated at 40% power for one minute to disperse all the solid pieces into solution to form a thick dispersed mass. This was followed by 5 minutes of sonication at 50% power to produce a homogeneous solution with the consistency of milk. The contents from all three tubes were added to a lyophilization jar and 450 mg of croscarmellose was added followed by light sonication (40% power for 30 seconds) to disperse all the components into solution. The blend was then frozen with dry ice acetone and lyophilized.

The lyophilized off-white solid was milled in a coffee grinder and a 2.75-gram portion was wet granulated with 0.11 gram calcium carbonate using a 10% polyvinylpyrollidone K-30 (Spectrum, New Brunswick, NJ) solution in 91 % isopropanol/water. After drying, the granulation was milled with silicon dioxide and passed through a #10 sieve. Granules were packed into each of four "000" capsules. To check the dissolution time, 100 mg of granulated material was added to a "00" capsule and added to 200 mL of warm water. With gentle inversion, all the particles were dispersed in 12 minutes and a milky solution was formed.

*Commercial Spironolactone Solid Formulation.* Commercial spironolactone tablets (100 mg, Mylan Pharmaceuticals) were purchased from a local pharmacy.

Each of the three formulations was administered to each of four dogs in a crossover study with a one week washout period between doses. Plasma samples were analyzed for spironolactone and its metabolite, canrenone, at MDS Pharma Services (St, Laurent, Quebec Canada) using an LC/MS/MS assay following a procedure especially established for dogs with an analytical range of 5 - 500 ng/mL. Samples outside the upper range were diluted before assay.

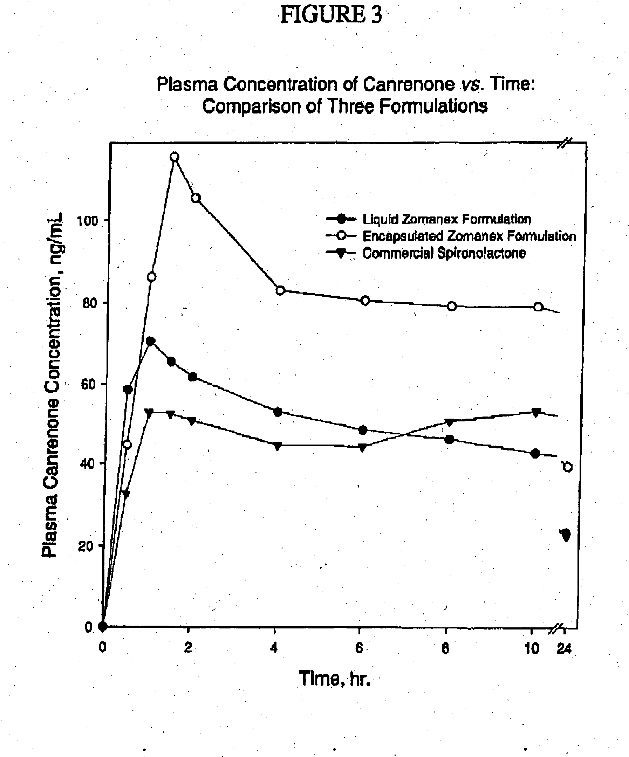

As shown in Figure 3, the solid lysolecithin sterol formulation resulted in higher plasma canrenone concentrations at all time points (except 0.5 hr.), which is reflected in the area under the curve shown in the table below. Thus, the area under the curve for the solid lysolecithin sterol formulation was 65% greater (p = 0.02) than that for the corresponding liquid formulation and for the commercial spironolactone (p = 0.12).

| Formulation | Mean AUC ± SEM ng/mL h⁻¹ |
|---|---|
| Liquid Lysolecithin Sterol Formulation (A) | 1000.1 ± 141.7 |
| Solid Lysolecithin Sterol Formulation^{‡} (B) | 1648.3 ± 267.7 |
| Solid Commercial Sprionolactone (C) | 995.7 ± 230.9 |

| | |
|---|---|
| ^{‡} Only formulation that contained calcium carbonate A *vs,* B, p = 0.02; A *vs.* C, p = 0.98; B *vs.* C, p = 0.12 | |

For each formulation the coefficient of variation of the plasma canrenone concentration was determined at each time point for the four dogs, and the mean was calculated. As shown in the table below, the mean coefficient of variation was lowest for the liquid lysolecithin sterol formulation, 15.2%, and statistically lower than that for the solid lysolecithin sterol formulation and commercial spironolactone, 20.4% (p = 0.05) and 27.8% (p = 0.004), respectively.

| Formulation | CV, % |
|---|---|
| Liquid Lysolecithin Sterol Formulation (A) | 15.2 |
| Solid Lysolecithin Sterol Formulation (B) | 20.4 |
| Solid Commercial Sprionalactone (C) | 27.8 |

| | |
|---|---|
| A *vs*. B, p = 0.06; A *vs*. C, p = 0.004; B *vs*, C, p = 0.05 | |

Taken together, these data show that the lysolecithin sterol formulation either in the liquid form or solid form provides less absorption variability when compared to the commercial preparation. Surprisingly, the solid lysolecithin sterol formulation provided greater absorption than that provided by the corresponding liquid preparation. Besides the different physical state, the only difference between the liquid and solid lysolecithin sterol formulation was the presence of calcium carbonate in the solid. There are many points where calcium may influence the uptake process, including but not limited to, (1) stabilizing the formulation as it passes through the stomach; (2) promoting pancreatic phospholipase A₂ activity in the small intestinal system; (3) promoting fusion of the hydrated sterol/lysolecithin/drug formulation with the small intestinal cell; or (4) activating a calcium-dependent process that promotes uptake of lipids by the small intestinal cell.

### EXAMPLE 4

To verify the flexibility of the formulation system and the effect of calcium carbonate on drug uptake, amiodarone was chosen as another test drug. The solid hydrochloride salt of amiodarone was used in the commercial preparation, but when this solid was neutralized the resulting free base was an oil. Therefore, this drug provided an opportunity to test the ability of the lysolecithin sterol combination to disperse an oil in a solid sterol lysolecithin matrix that could be delivered as a conventional tablet or capsule.

*Liquid Formulation.* Amiodarone hydrochloride was converted to the free base oil by LKT Laboratories (St. Paul, MN). Amiodarone (200 mg), soy sterols (75 mg, Archer Daniels Midland, Decatur, IL) and soy lysolecithin (300 mg Solae Corporation, Fort Wayne IN) were dissolved in chloroform with gentle warming and the solvent was removed under a stream of nitrogen, followed by exhaustive pumping. On the day of the experiment, water (10 mL) was added to the glass tube and the mixture was sonicated for 30 seconds on ice using a Branson Digital Sonifier (Model S450D), equipped with a 1/8" tapered tip to form a milky emulsion, which was delivered to the dog with a syringe. Water was added to the syringe and the washing was administered to the dog.

*Solid Formulation Derived From the Liquid Formulation.* Amiodarone oil (800 mg) was carefully poured into each of three 30-mL heavy-wall glass tubes followed by 4 mL of chloroform with gentle swirling. To the homogeneous solution, 300 mg of soy sterols and 1200 mg of soy lecithin were added with warming. To prepare the lyophilized emulsion of the drug/plant sterol/lysolecithin blend, the same steps were followed as that described in Example 3.

The lyophilized off-white solid was slightly sticky and to eliminate this property and to increase its bulk density, a 3.05 gm portion was dry blended with calcium carbonate (0.10 gm) and silicon dioxide (0.10 gm) in a plastic container. The container was vigorously shaken and the solid was milled repeatedly with short bursts. The stickiness disappeared and four capsules were filled with 575 mg of dry blended small solid particles. To check the dissolution time, 100 mg of material was added to a "00" capsule and added to 200 mL of warm water. With gentle inversion, all the particles were dispersed in 10 minutes.

*Commercial Amiodarone Solid Formulation.* Commercial amiodarone(Pacerone^{®}) tablets (200 mg, Upsher Smith Pharmaceuticals) were purchased from a local pharmacy.

Each of the three formulations was administered to each of four dogs in a crossover study with a one week washout period between doses. Serum samples were analyzed for amiodarone and its metabolite, N-desethyl amiodarone, at Ralston Analytical Services (St. Louis, MO) using an HPLC assay following a procedure used for dogs. N-Desethyl amiodarone was not used in the analysis since its concentration was only slightly above the level of detection of the assay system.

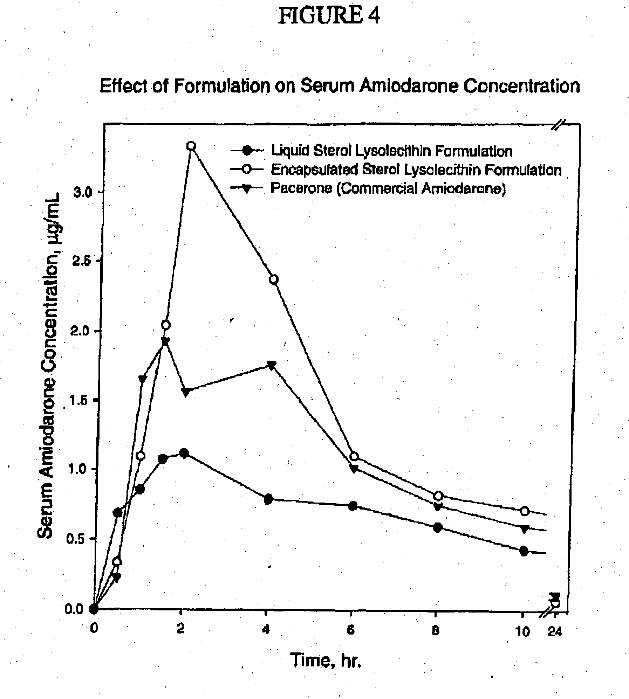

As shown in Figure 4, the solid lysolecithin sterol formulation resulted in higher plasma amiodarone concentrations at all time points, which is reflected in the area under the curve shown in the table below. Thus, the area under the curve for the solid lysolecithin sterol formulation was 2-fold greater (p = 0.005) than that for the corresponding liquid formulation and 0.25-fold greater than that for Pacerone^{®} (p = 0.08).

| Formulation | Mean AUC ± SEM µg/mL h⁻¹ |
|---|---|
| Liquid Lysolecithin Strerol Formulation (A) | 11.0 ± 2.5 |
| Solid Lysolecithin Sterol Formulation^{‡} (B) | 20.7 ± 3.3 |
| Solid Commercial Sprionolactone (C) | 16.5 ± 3.4 |

| | |
|---|---|
| ^{‡} Only formulation that contained calcium carbonate A *vs.* B, p = 0.005; A *vs.* C, p = 0.11; B *vs*. C, p = 0.08 | |

This experiment shows that the formulation method described herein is flexible and adaptable to drugs whose physical state is an oil, a difficult form to formulate in a solid dosage form. The area under the curve of the encapsulated sterol lysolecithin drug was 25% greater than that for Pacerone^{®}, a value that approached statistical significance and that indicates that not only can an oil be formulated in this manner, but that the uptake of the drug may be superior to that for the commercial preparation from the drug hydrochloride salt.

In addition, the results presented here also verify the importance of calcium carbonate for enhancing the uptake of drug using the sterol lysolecithin formulation system. In this case, the calcium carbonate-containing solid formulation produced a statistically significant 2-fold increase in the area under the curve when compared to that for the corresponding liquid formulation.

## Claims

1. A drug delivery composition that enhances the oral absorption of normally difficulty soluble hydrophobic oil or crystalline drug actives, comprising:
an emulsifier, said emulsifier being a phospholipid, a lecithin, a lysolecithin or combinations thereof;
a plant derived sterol (stand) or ester derived from the sterol (stanol);
a drug active effective amount of a hydrophobic drug, in which the weight ratio of the emulsifier to the plant Sterol drug combination is from 0.1 to 3.0; and
**characterized in that** the composition further comprises an antacid.

2. The composition of claim 1 wherein the antacid is calcium carbonate.

3. The composition of claim 2 wherein the weight ratio of calcium carbonate in the mixture is from 0.005 to 0.10.

4. The drug delivery composition of claim 1 wherein the plant derived sterol (stanol) is a plant derived sterol (stanol) ester, derived from a vegetable oil source.

5. The composition of claim 1 wherein the weight ratio is 1.0.

6. The composition of claim 1 wherein the weight ratio is 1.5.

7. The composition of claim 1 wherein the drug delivery composition includes as an additional hydrophobic compound, vitamin E.

8. The method of preparing a drug delivery system for enhancing the oral absorption of normally difficulty soluble hydrophobic drug actives, comprising:
Mixing an emulsifier(s), said emulsifier(s) being a phospholipid, a lecithin, a Lysolecithin or mixtures thereof, with a plant derived sterol. (stanol) or esters derived from plant sterol (stanol) in which the fatty acid ester moiety is derived from a vegetable oil, and a drug active, with a non-polar solvent;
removing the solvent to leave a solid residue of the mixed components;
adding water to the solid residue of the mixed components at a temperature less than the decomposition temperature of any one of the mixed components;
homogenizing the aqueous mixture;
drying the homogenized mixture;
providing the dried solid residue of the mixed components in a solid pharmaceutical carrier format, in which the weight ratio of the emulsifier to the plant sterol drug combination is from 0.1 to 3.0; and
**characterized in that** an antacid is added to the mixture.

9. The method of claim 8 wherein the emulsifier is a compound that is approved for use in foods or for pharmaceutical applications.

10. The method of claim 8 wherein the non-polar organic solvent is selected from the group consisting of ethyl acetate and heptane.

11. The method of claim 8 wherein the non-polar organic solvent is at its boiling point.

12. The method of claim 8 wherein the non-polar organic solvent is removed by elevating the temperature above the solvent's boiling point.

13. The method of claim 8 wherein the dried solid residue of the mixed components is dispersed in water with vigorous stirring at a temperature less than the decomposition temperature of any of the mixed components.

14. The method of claim 8 wherein an additional step, prior to final drying includes homogenizing of the water dispersed mixed components.

15. The method of claim 8 wherein the solid formed after solvent removal is pulverized in an appropriate mill, grinder or processor to produce a dispersible powder.

16. The method of claim 8 wherein the non-polar organic solvent is selected from the group consisting of heptane, chloroform, dichloromethane and isopropanol.

17. The method of claim 8 wherein the solvent removal continues until a solid residue that contains less than 0.5% solvent is provided,

18. The method of claim 8 wherein the solid formed after solvent removal is pulverized to produce a dispersible powder.

19. The method of claim 18 wherein the dispersible powder is added with vigorous stirring to water at a temperature that is less than the decomposition temperature of any of the mixed components.

20. The method of claim 8 wherein water is introduced directly to the un-pulverized dried solid residue.

21. The method of claim 20 wherein the water is at a temperature that is less than the decomposition temperature of any one of the mixed components.

22. The method of claim 8 wherein the aqueous mixture is homogenized in a homogenizer selected from the group consisting of a Gaulin homogenizer, a French press, a sonicator, and a microfludizer.

23. The method of claim 8 wherein the homogenized aqueous mixture is dried in a drier selected from the group consisting of spray driers and lyophilizers.

24. The method of claim 23, wherein a drying aid selected from the group consisting of starch, silicon dioxide and calcium silicate is added.

25. The method of claim 8 wherein the antacid is calcium carbonate.

26. The method of claim 25, wherein the antacid is added such that its weight ratio in the drug, plant sterol lecithin blend is between 0.005 and 0.1, with a preferred ratio of 0.035.

27. The method of claim 25 wherein the solid is converted into a tablet or capsule.

28. The method of claim 25 wherein the solid is added to a beverage or medical food product.

29. A solid product that is formed from the method of claim 18 by subjecting the material to compression or extrusion for at least 15 seconds at a pressure of at least 100 psig.

30. A solid product that is formed from the method of claim 23 by subjecting the material to a compression or extrusion for at least 15 seconds at a pressure of at least 100 psig.

## Patentansprüche

1. Arzneimittelzuführungszusammensetzung, die die orale Absorption von normalerweise schwer löslichem hydrophobem Öl oder kristallinen Arzneimittelwirkstoffen verbessert, die Folgendes umfasst:
einen Emulgator, wobei der genannte Emulgator ein Phospholipid, ein Lecithin, ein Lysolecithin oder eine Kombination davon ist;
ein pflanzliches Sterol (Stanol) oder von dem Sterol (Stanol) gewonnener Ester;
eine als Arzneimittelwirkstoff wirksame Menge eines hydrophoben Arzneimittels, wobei das Gewichtsverhältnis zwischen Emulgator und Pflanzensterol-Arzneimittel-Kombination 0,1 bis 3,0 beträgt; und
**dadurch gekennzeichnet, dass** die Zusammensetzung ferner ein Antazidum umfasst.

2. Zusammensetzung nach Anspruch 1, wobei das Antazidum Calciumcarbonat ist.

3. Zusammensetzung nach Anspruch 2, wobei der Gewichtsanteil von Calciumcarbonat in dem Gemisch 0,005 bis 0,10 beträgt.

4. Arzneimittelzuführungszusammensetzung nach Anspruch 1, wobei das pflanzliche Sterol (Stanol) ein von einer Pflanzenölquelle gewonnener pflanzlicher Sterol-(Stanol)-Ester ist.

5. Zusammensetzung nach Anspruch 1, wobei der Gewichtsanteil 1,0 beträgt.

6. Zusammensetzung nach Anspruch 1, wobei der Gewichtsanteil 1,5 beträgt.

7. Zusammensetzung nach Anspruch 1, wobei die Arzneimittelzuführungszusammensetzung als zusätzliche hydrophobe Verbindung Vitamin E enthält.

8. Verfahren zur Herstellung eines Arzneimittelzuführungssystems zum Verbessern der oralen Absorption von normalerweise schwer löslichen hydrophoben Arzneimittelwirkstoffen, das Folgendes beinhaltet:
Mischen von einem oder mehreren Emulgatoren, wobei der/die Emulgator(en) ein Phospholipid, ein Lecithin, ein Lysolecithin oder Mischungen davon ist/sind, mit einem pflanzlichen Sterol (Stanol) oder mit von pflanzlichem Sterol (Stanol) gewonnenen Estern, wobei der Fettsäureesteranteil von einem Pflanzenöl gewonnen wurde, und einen Wirkstoff, mit einem nichtpolaren Lösungsmittel;
Entfernen des Lösungsmittels, um einen Feststoffrest der gemischten Komponenten zu hinterlassen;
Zugeben von Wasser zu dem festen Rest der gemischten Komponenten mit einer Temperatur von weniger als der Zersetzungstemperatur einer beliebigen der gemischten Komponenten;
Homogenisieren des wässrigen Gemischs;
Trocknen des homogenisierten Gemischs;
Bereitstellen des getrockneten Feststoffrests der gemischten Komponenten in einem festen pharmazeutischen Trägerformat, in dem der Gewichtsanteil des Emulgators an der Pflanzensterol-Arzneimittelkombination 0,1 bis 3,0 beträgt; und
**dadurch gekennzeichnet, dass** dem Gemisch ein Antazidum zugegeben wird.

9. Verfahren nach Anspruch 8, wobei der Emulgator eine Verbindung ist, die für den Einsatz in Lebensmittel oder für pharmazeutische Anwendungen zugelassen ist.

10. Verfahren nach Anspruch 8, wobei das nichtpolare organische Lösungsmittel aus der Gruppe bestehend aus Ethylacetat und Heptan ausgewählt ist.

11. Verfahren nach Anspruch 8, wobei das nichtpolare organische Lösungsmittel auf seinem Siedepunkt ist.

12. Verfahren nach Anspruch 8, wobei das nichtpolare organische Lösungsmittel durch Erhöhen der Temperatur über den Siedepunkt des Lösungsmittels hinaus entfernt wird.

13. Verfahren nach Anspruch 8, wobei der getrocknete Feststoffrest der gemischten Komponenten in Wasser unter starkem Rühren bei einer Temperatur unter der Zersetzungstemperatur aller gemischten Komponenten dispergiert wird.

14. Verfahren nach Anspruch 8, wobei ein zusätzlicher Schritt, vor dem endgültigen Trocknen, das Homogenisieren der in Wasser dispergierten gemischten Komponenten beinhaltet.

15. Verfahren nach Anspruch 8, wobei der nach dem Entfernen des Lösungsmittels gebildete Feststoff in einer geeigneten Mühle, einem Zerkleinerer oder Prozessor pulverisiert wird, um ein dispergierbares Pulver zu erzeugen.

16. Verfahren nach Anspruch 8, wobei das nichtpolare organische Lösungsmittel ausgewählt ist aus der Gruppe bestehend aus Heptan, Chloroform, Dichlormethan und Isopropanol.

17. Verfahren nach Anspruch 8, wobei das Entfernen des Lösungsmittels fortgesetzt wird, bis ein Feststoffrest vorliegt, der weniger als 0,5 % Lösungsmittel enthält.

18. Verfahren nach Anspruch 8, wobei der nach dem Entfernen des Lösungsmittels gebildete Feststoff pulverisiert wird, um ein dispergierbares Pulver zu erzeugen.

19. Verfahren nach Anspruch 18, wobei das dispergierbare Pulver unter kräftigem Rühren Wasser bei einer Temperatur unter der Zersetzungstemperatur von einer der gemischten Komponenten zugegeben wird.

20. Verfahren nach Anspruch 8, wobei Wasser direkt in den unpulverisierten getrockneten Feststoffrest eingeleitet wird.

21. Verfahren nach Anspruch 20, wobei das Wasser auf einer Temperatur unter der Zersetungstemperatur aller gemischten Komponenten ist.

22. Verfahren nach Anspruch 8, wobei das wässrige Gemisch in einem Homogenisierer homogenisiert wird, der aus der Gruppe bestehend aus einem Gaulin-Homogenisierer, einer French-Presse, einem Beschaller und einem Mikrofluidisierer ausgewählt ist.

23. Verfahren nach Anspruch 8, wobei das homogenisierte wässrige Gemisch in einem Trockner getrocknet wird, der aus der Gruppe bestehend aus Sprühtrocknern und Lyophilisierem ausgewählt ist.

24. Verfahren nach Anspruch 23, wobei eine Trocknungshilfe zugegeben wird, die aus der Gruppe bestehend aus Stärke, Siliciumdioxid und Calciumsilikat ausgewählt ist.

25. Verfahren nach Anspruch 8, wobei das Antazidum Calciumcarbonat ist.

26. Verfahren nach Anspruch 25, wobei das Antazidum so zugegeben wird, dass sein Gewichtsanteil an der Arzneimittel-Pflanzensterollecithin-Mischung 0,005 bis 0,1, vorzugsweise 0,035 beträgt.

27. Verfahren nach Anspruch 25, wobei der Feststoff in eine Tablette oder Kapsel umgewandelt wird.

28. Verfahren nach Anspruch 25, wobei der Feststoff einem Getränk oder einem medizinischen Lebensmittelprodukt zugegeben wird.

29. Feststoffprodukt, das mit dem Verfahren nach Anspruch 18 gebildet wird, durch Unterziehen des Materials einer Kompression oder Extrusion für wenigstens 15 Sekunden bei einem Druck von wenigstens 100 psig.

30. Feststoffprodukt, das mit dem Verfahren nach Anspruch 23 gebildet wird, durch Unterziehen des Materials einer Kompression oder Extrusion für wenigstens 15 Sekunden bei einem Druck von wenigstens 100 psig.

## Revendications

1. Formule pour la libération/délivrance de médicaments qui améliore l'absorption orale de principes actifs médicamenteux [type] huile hydrophobe ou produit cristallisé normalement difficile[ment] solubles, comprenant :
un émulsifiant, ledit émulsifiant étant un phospholipide, une lécithine, une lysolécithine ou une combinaison de ceux-ci ;
un stérol provenant de plantes (stanol) ou un ester dérivé de ce stérol (stanol) ;
une quantité pharmaceutiquement active efficace d'un médicament hydrophobe, dans laquelle le rapport pondéral de l'émulsifiant à la combinaison médicamenteuse stérol végétal va de 0,1 à 3,0 ; et
**caractérisée en ce que** la formule comprend en outre un antiacide.

2. Formule selon la revendication 1, l'antiacide étant le carbonate de calcium.

3. Formule selon la revendication 2, le rapport pondéral du carbonate de calcium dans le mélange allant de 0,005 à 0,10.

4. Formule pour la délivrance de médicaments selon la revendication 1, le stérol provenant de plantes (stanol) étant un ester de stérol provenant de plantes (stanol), provenant d'une source huile végétale.

5. Formule selon la revendication 1, le rapport pondéral étant de 1,0.

6. Formule selon la revendication 1, le rapport pondéral étant de 1,5.

7. Formule selon la revendication 1, la formule pour la délivrance de médicaments comprenant en tant que composé hydrophobe supplémentaire la vitamine B.

8. Méthode de préparation d'un système de délivrance de médicaments pour améliorer l'absorption orale de principes actifs médicamenteux hydrophobes normalement difficile[ment] solubles, comprenant :
le mélange d'un émulsifiant(s), le(s)dit(s) émulsifiant(s) étant un phospholipide, une lécithine, une lysolécithine ou des mélanges de ceux-ci, avec un stérol provenant de plantes (stanol) ou des esters dérivés d'un stérol végétal (stanol) dans lesquels la partie ester d'acide gras est dérivée d'une huile végétale, et un principe actif médicamenteux, avec un solvant non polaire ;
l'enlèvement du solvant, laissant un résidu solide des composants mélangés ;
l'addition d'eau au résidu solide des composants mélangés à une température inférieure à la température de décomposition de n'importe lequel des composants mélangés ;
l'homogénéisation du mélange aqueux ;
le séchage du mélange homogénéisé ;
la fourniture du résidu solide séché des composants mélangés sous une forme véhicule pharmaceutique solide, dans laquelle le rapport pondéral de l'émulsifiant à la combinaison médicamenteuse stérol végétal va de 0,1 à 3,0 ; et
**caractérisée en ce qu'**un antiacide est ajouté au mélange.

9. Méthode selon la revendication 8, l'émulsifiant étant un composé qui est approuvé pour utilisation dans des aliments ou pour des applications pharmaceutiques.

10. Méthode selon la revendication 8, le solvant organique non polaire étant sélectionné parmi le groupe consistant en l'acétate d'éthyle et l'heptane.

11. Méthode selon la revendication 8, le solvant organique non polaire étant à son point d'ébullition.

12. Méthode selon la revendication 8, le solvant organique non polaire étant enlevé en élevant la température au-dessus du point d'ébullition du solvant.

13. Méthode selon la revendication 8, le résidu solide séché des composants mélangés étant dispersé dans de l'eau en agitant vigoureusement à une température inférieure à la température de décomposition de n'importe lequel des composants mélangés.

14. Méthode selon la revendication 8, une étape supplémentaire avant le séchage final comprenant l'homogénéisation des composants mélangés dispersés dans de l'eau.

15. Méthode selon la revendication 8, le solide formé après enlèvement du solvant étant pulvérisé dans un broyeur, pulvérisateur ou machine de traitement appropriée pour produire une poudre dispersible.

16. Méthode selon la revendication 8, le solvant organique non polaire étant sélectionné parmi le groupe consistant en l'heptane, le chloroforme, le dicholorométhane et l'isopropanol.

17. Méthode selon la revendication 8, l'enlèvement du solvant étant poursuivi jusqu'à obtention d'un résidu solide contenant moins de 0,5 % de solvant.

18. Méthode selon la revendication 8, le solide formé après enlèvement du solvant étant pulvérisé pour produire une poudre dispersible.

19. Méthode selon la revendication 18, la poudre dispersible étant ajoutée à de l'eau en agitant vigoureusement à une température inférieure à la température de décomposition de n'importe lequel des composants mélangés.

20. Méthode selon la revendication 8, l'eau étant introduite directement avec le résidu solide séché non pulvérisé.

21. Méthode selon la revendication 20, l'eau étant à une température inférieure à la température de décomposition de n'importe lequel des composants mélangés.

22. Méthode selon la revendication 8, le mélange aqueux étant homogénéisé dans un homogénéisateur sélectionné parmi le groupe consistant en un homogénéisateur Gaulin, une cafetière à piston, un sonicateur et un microfluidisateur.

23. Méthode selon la revendication 8, le mélange aqueux homogénéisé étant séché dans un séchoir sélectionné parmi le groupe consistant en séchoirs atomiseurs et lyophilisateurs.

24. Méthode selon la revendication 23, un agent dessiccatif sélectionné parmi le groupe consistant en l'amidon, le dioxyde de silicium et le silicate de calcium étant ajouté.

25. Méthode selon la revendication 8, l'antiacide étant le carbonate de calcium.

26. Méthode selon la revendication 25, l'antiacide étant ajouté de manière à ce que son rapport pondéral dans le médicament mélange lécithine stérol végétal soit compris entre 0,005 et 0,1, avec un rapport préféré de 0,035.

27. Méthode selon la revendication 25, le solide étant transformé en un comprimé ou une gélule.

28. Méthode selon la revendication 25, le solide étant ajouté à une boisson ou à un aliment thérapeutique.

29. Produit solide formé selon la méthode de la revendication 18 en soumettant le matériau à une compression ou une extrusion pendant au moins 15 secondes à une pression d'au moins 100 psi (6,9 bars).

30. Produit solide formé selon la méthode de la revendication 23 en soumettant le matériau à une compression ou une extrusion pendant au moins 15 secondes à une pression d'au moins 100 psi (6,9 bars).
